(19) Europäisches Patentamt
European Patent Office
Office européen des brevets

(11) Veröffentlichungsnummer : **0 090 769**
**B1**

(12) **EUROPÄISCHE PATENTSCHRIFT**

(45) Veröffentlichungstag der Patentschrift : **20.08.86**

(21) Anmeldenummer : **83810114.5**

(22) Anmeldetag : **21.03.83**

(51) Int. Cl.[4] : **C 07 D471/06**// A01N43/90 ,(C07D471/06, 221:00, 209:00)

(54) **Verfahren zur Herstellung von 1,2,5,6-Tetrahydro-4H-pyrrolo(3,2,1-ij)-chinolin-4-on und neue 5-Halogen-1,2,3-(1,2-dihydropyrrolo)-4-chinolone als Zwischenprodukte zur Durchführung des Verfahrens.**

(30) Priorität : **25.03.82 CH 1839/82**

(43) Veröffentlichungstag der Anmeldung : **05.10.83 Patentblatt 83/40**

(45) Bekanntmachung des Hinweises auf die Patenterteilung : **20.08.86 Patentblatt 86/34**

(84) Benannte Vertragsstaaten : **AT BE CH DE FR GB IT LI NL SE**

(56) Entgegenhaltungen : **GB-A- 1 394 373**

(73) Patentinhaber : **CIBA-GEIGY AG**
**Klybeckstrasse 141**
**CH-4002 Basel (CH)**

(72) Erfinder : **Martin, Pierre, Dr.**
**Meisenweg 38**
**CH-4310 Rheinfelden (CH)**

**EP 0 090 769 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von 1,2,5,6-Tetrahydro-4H-pyrrolo[3,2,1-ij]chinolin-4-on und neue 5-Halogen-1,2,3-(1,2-dihydropyrrolo)-4-chinolone, die bei der Durchführung dieses Verfahrens als Zwischenprodukte durchlaufen werden.

Das 1,2,5,6-Tetrahydro-4H-pyrrolo [3,2,1-ij] chinolin-4-on entspricht der Formel I

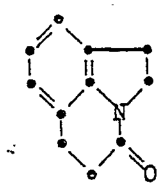

(I)

und ist auch unter dem Trivialnamen 5-Lilolidon bekannt. Die Verbindung kann als systemisches Fungizid zum Schutz von Kulturpflanzen, z. B. Reis, gegen den Befall durch phytopathogene Mikroorganismen und die dadurch verursachten Pflanzenkrankheiten verwendet werden. (vgl. GB-PS 1.394.373).

4-Lilolidon wurde bisher durch eine intramolekulare Friedel-Crafts Alkylierung aus N-(β-Chlorpropionyl)-indolin hergestellt. (vgl. J. Chem. Soc. 1518, (1964), GB-PS 1.394.373 und J. Agric. Food Chem. *29*, 576 (1981)). Bei diesen Verfahren wird ein grosser Ueberschuss an Aluminiumchlorid, hohe Reaktionstemperaturen oder lange Reaktionszeiten benötigt. Das Verfahren ist auch insofern nachteilig, als sich die Reaktionswärme nur schwer abführen lässt und die Abtrennung von Nebenprodukten sowie die Aufarbeitung des Endproduktes sehr langwierig und aufwendig sind. Aus diesem Grund ist das Verfahren für eine kostengünstige Herstellung von 4-Lilolidon in technischem Massstab nicht geeignet.

Es war daher das Ziel der vorliegenden Erfindung, das 4-Lilolidon auf einfache und wirtschaftliche Weise in guten Ausbeuten und in hoher Reinheit zugänglich zu machen.

Gemäss vorliegende Erfindung wird vorgeschlagen, 4-Lilolidon in der Weise herzustellen, dass man ein Halogenacetylindolin der Formel II

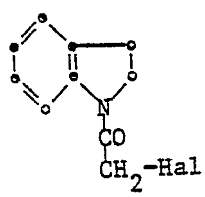

(II)

in welcher Hal Chlor oder Brom bedeutet, mit einem aus einem N,N-disubstituierten Formamid der Formel III

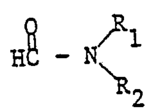

(III)

in welcher $R_1$ $C_1$-$C_4$-Alkyl oder Phenyl und $R_2$ $C_1$-$C_4$-Alkyl bedeutet, und einem Säurehalogenid gebildeten Additionsprodukt zu einem 5-Halogen-1,2,3-(1,2-dihydropyrrolo)-4-chinolon der Formel IV

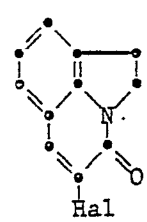

(IV)

in welcher Hal die oben angegebene Bedeutung hat, umsetzt und dieses durch katalytische Hydrierung in 4-Lilolidon der Formel I überführt.

Die 5-Halogen-1,2,3-(1,2-dihydropyrrolo)-4-chinolone der Formel IV sind neue Verbindungen und ebenfalls Gegenstand der Erfindung.

Die Umsetzung eines Halogenacetylindolins der Formel II mit dem aus einem N,N-disubstituierten Formamid der Formel III und einem Säurehalogenid gebildeten Additionsprodukt wird zweckmässig in einem inerten Lösungsmittel vorgenommen. Als solche Lösungsmittel eignen sich insbesondere halogenierte aliphatische oder aromatische Kohlenwasserstoffe, wie Chloroform, Dichlormethan, Dichlorethan, Kohlenstofftetrachlorid, Chlorbenzol, Dichlorbenzole, Toluol und Xylol. Ferner kann auch überschüssiges N,N-disubstituiertes Formamid der Formel III und vor allem überschüssiges Säurechlorid als Lösungsmittel verwendet werden. Bevorzugte Lösungsmittel sind 1,2-Dichlorethan, Chloroform und insbesondere Toluol. Besonders vorteilhaft ist auch die Verwendung von überschüssigen Phosphoroxychlorid als Lösungsmittel.

Als Säurehalogenide kommen allgemein solche in Betracht, die mit einem N,N-disubstituierten Formamid der Formel III unter Bildung eines Vilsmeier-Komplexes reagieren können. Geeignete Säurehalogenide sind beispielsweise Phosphortrichlorid, Phosphortribromid, Phosphorpentachlorid, Phosphoroxychlorid, Phosphoroxybromid, Phosgen, Carbonyldibromid, Carbonyldifluorid, Oxalylchlorid, Heptachlorbuttersäurechlorid, Thionylchlorid und Thionylbromid. Ferner können auch Derivate von einigen der vorgenannten Säurechloride verwendet werden, wie z. B. 2,2,2-Trichlor-1,3-dioxa-2-phosphaindan und 2,2-Dichlor-1,3-dioxaindan, die als Derivate des Phosphoroxychlorids und Phosgens aufgefasst werden können. Bevorzugte Säurehalogenide sind Phosphoroxychlorid und Phosgen.

Geeignete N,N-disubstituierte Formamide der Formel III sind bsp. N,N-Dimethylformamid, N,N-Diethylformamid, N,N-Dipropylformamid, N,N-Dibutylformamid, N-Butyl-N-methylformamid und N-Methyl-N-phenylformamid (N-Formyl-N-methylanilin).

Bevorzugte N,N-disubstituierte Formamide der Formel II sind N,N-Dimethylformamid und N-Methyl-N-phenylformamid. Besonders bevorzugt ist N,N-Dimethylformamid.

Das Säurehalogenid wird bei der Durchführung des Verfahrens in einem organischen Lösungsmittel in einer Menge von mindestens 2 Mol pro Mol N-Halogenacetylindolin der Formel II eingesetzt. Vorzugsweise wird das Säurehalogenid bei der Durchführung des Verfahrens in einem organischen Lösungsmittel in einer Menge von 2.5-5.0 Mol pro Mol N-Halogenacetylindolin der Formel II eingesetzt.

Die Umsetzung eines Halogenacetylindolins der Formel II mit dem aus einem N,N-disubstituierten Formamid der Formel III und einem Säurehalogenid gebildeten Additionsprodukt kann in der Weise durchgeführt werden, dass man zunächst aus dem N,N-disubstituierten Formamid und dem Säurehalogenid das Additionsprodukt herstellt und danach das Halogenacetylindolin der Formel II zugibt. Die Umsetzung kann aber auch so durchgeführt werden, dass man das aus einem N,N-disubstituierten Formamid der Formel III und einem Säurehalogenid gebildete Additionsprodukt dem Halogenacetylindolin der Formel II zufügt. Ferner kann die Umsetzung auch in vorteilhafterweise so vorgenommen werden, dass man eine Mischung eines Halogenacetylindolins der Formel II und eines N,N-disubstituierten Formamids der Formel III vorlegt und das Säurehalogenid in diese Mischung einträgt und so die Bildung des Additionsprodukts aus N,N-disubstituierten Formamid der Formel III und dem Säurehalogenid *in situ* durchführt.

Die Reaktionstemperaturen liegen in der Regel zwischen 40° und 100 °C. Die Reaktion ist im allgemeinen innerhalb weniger Stunden beendet. Besonders vorteilhaft wird die Umsetzung bei Temperaturen zwischen 50 und 75 °C durchgeführt. Bei diesen Reaktionstemperaturen ergeben sich Reaktionszeiten von 1-2 Stunden. Unter diesen bevorzugten, relativ milden Bedingungen verläuft die Reaktion im allgemeinen ohne nennenswerte Bildung von Nebenprodukten.

Nach der Umsetzung des Halogenacetylindolins der Formel II mit dem aus einem N,N-disubstituierten Formamid der Formel III und einem Säurehalogenid gebildeten Additionsprodukt kann das Reaktionsgemisch auf einfache Weise, z. B. durch Eingiessen in wässrige Natriumhydroxydlösung, aufgearbeitet werden. Man erhält so eine Suspension des 5-Halogen-1,2,3-(1,2-dihydropyrrolo)-4-chinolons der Formel IV, aus der das Produkt auf einfache Weise durch Filtration und Trocknen gewonnen werden kann. Falls die Umsetzung des Halogenacetylindolins der Formel II mit dem aus einem N,N-disubstituierten Formamid der Formel III und einem Säurehalogenid gebildeten Additionsprodukt in Chloroform durchgeführt wurde, erhält man nach dem Eingiessen des Reaktionsgemisches in wässrige Natronlauge eine 2-phasige Mischung, in der das gewünschte 5-Halogen-1,2,3-(1,2-dihydropyrrolo)-4-chinolon der Formel IV als Lösung in der organischen Phase vorliegt. Durch Abtrennung der organischen Phase und Abdestillieren des Lösungsmittel erhält man das gewünschte 5-Halogen-1,2,3-(1,2-dihydropyrrolo)-4-chinolons der Formel IV.

Die katalytische Hydrierung der 5-Halogen-1,2,3-(1,2-dihydropyrrolo)-4-chinolone der Formel IV, die unter Abspaltung des 5-Halogens verläuft, wird zweckmässig in einem inerten organischen Lösungsmittel und zur Bindung des gebildeten Halogenwasserstoffs in Gegenwart einer Base durchgeführt. Als inerte Lösungsmittel kommen insbesondere aliphatische und aromatische Kohlenwasserstoffe, wie Cyclohexan, Toluol, Xylol, sowie niedere aliphatische Carbonsäuren, insbesondere Essigsäure, und ferner Alkohole, wie Methanol, Ethanol und Isopropanol, in Betracht. Als Basen, in deren Gegenwart die katalytische Hydrierung durchgeführt wird, werden vorteilhaft Alkali- und Erdalkalimetallhydroxyde, -carbonate und -hydrogencarbonate, sowie Ammoniak oder Amine verwendet. Ferner können als Basen auch Alkalimetallacetate, insbesondere Natriumacetat, verwendet werden.

Als weitere Basen, in deren Gegenwart die katalytische Hydrierung der 5-Halogen-1,2,3-(1,2-dihydropyrrolo)-4-chinolone der Formel IV vorgenommen werden kann, seien beispielsweise Natriumhydroxyd, Kaliumhydroxyd, Ammoniak, Triethylamin und Pyridin genannt.

Als Katalysatoren für die katalytische Hydrierung der 5-Halogen-1,2,3-(1,2-dihydropyrrolo)-4-chinolone der Formel IV sind Edelmetalle der Gruppe VIII des periodischen Systems, insbesondere Nickel, Palladium und Platin, geeignet. Die Katalysatoren kommen in fein verteilter Form, beispielsweise als

Raney-Nickel, oder auf einem Träger beispielsweise Palladium auf Kohle oder Platin auf Kohle zur Anwendung.

Die katalytische Hydrierung der 5-Halogen-1,2,3-(1,2-dihydropyrrolo)-4-chinolone der Formel IV wird in der Regel bei Normaldruck oder leicht erhöhtem Druck durchgeführt. In der Praxis wird die katalytische Hydrierung vorteilhaft bei Drucken von 1-20 bar, vorzugsweise 3-5 bar durchgeführt.

Die Temperaturen, bei denen die katalytische Hydrierung durchgeführt werden kann, liegen im allgemeinen zwischen Raumtemperatur und 130 °C. Als besonders vorteilhaft haben sich Temperaturen von 40-75 °C erwiesen.

Nach beendigter Hydrierung erfolgt die Aufarbeitung des Reaktionsgemisches zweckmässig durch Abfiltrieren des Katalysators und Abdampfen des Lösungsmittels.

Mit dem erfindungsgemässen Verfahren wird es möglich, 4-Lilolidon ausgehend von Halogenacetylindolinen der Formel II in einer Ausbeute von etwa 90 % der Theorie herzustellen. Das Verfahren ist einfach durchführbar und daher auch für eine Herstellung von 4-Lilolidon in technischem Massstab gut geeignet. Die als Ausgangsmaterial benötigten Halogenacetylindoline der Formel II können auf einfache Weise ausgehend von Indolin durch Umsetzung mit Halogenacetylhalogeniden, insbesondere Halogenacetylchlorid, oder durch Umsetzung von Indol mit Halogenacetylchlorid und katalytische Hydrierung des erhaltenen N-Halogenacetylindols hergestellt werden.

Das erfindungsgemässe Verfahren wird durch die folgenden Beispiele näher erläutert.

## Beispiel 1

a) Herstellung von 5-Chlor-1,2,3-(1,2-dihydropyrrolo)-4-chinolon.

19,55 g (0,1 Mol) N-Chloracetylindolin werden portionsweise in eine Mischung von 150 ml (251,2 g ; 1,64 Mol) Phosphoroxychlorid und 20 ml (19,0 g ; 0,26 Mol) N,N-Dimethylformamid eingetragen. Nach beendigter Zugabe des N-Chloracetylindolins wird 1,75 Stunden auf 70-75 °C Innentemperatur erwärmt. Danach wird das überschüssige Phosphoroxychlorid unter vermindertem Druck bei 40 °C abgedampft. Der Rückstand wird in kalte Natriumhydroxydlösung (10 %ig) gegossen, wobei sich das Produkt abscheidet. Nach 1-stündigem Rühren wird der Niederschlag abfiltriert und getrocknet. Man erhält 20,05 g (97,8 % der Theorie) 5-Chlor-1,2,3-(1,2-Dihydropyrrolo)-4-chinolon in Form eines beigen Pulvers ; Schmelzpunkt 190-192 °C.

IR-Spektrum ($CHCl_3$) : 1 660, 1 640 (CO, C=C)$cm^{-1}$ · $H^1$-NMR-Spektrum (100 MHz, $CDCl_3$) : 3,33 (breites t, 2H), 4,30 (breites t, 2H), 6,95-7,30 (m, 3H), 7,85 (s, 1H) ppm. $^{13}C$-NMR-Spektrum ($CDCl_3$ : 156,6, 141,3, 130,4, 123,7, 116,5, 47,8 und 27,3 (alles s), sowie 134,7, 125,1 123,8 und 122,8 (alles d) ppm.

Das abgedampfte Phosphoroxychlorid ist rein und kann wieder verwendet werden. Das als Ausgangsmaterial benötigte N-Chloracetylindolin wird auf übliche Weise aus Indolin und Chloracetylchlorid hergestellt ; Schmelzpunkt 129-130 °C.

b) Herstellung von 4-Lilolidon

20,0 g (0,097 Mol) 5-Chlor-1,2,3-(1,2-dihydropyrrolo)-4-chinolon werden mit 8,0 g Natriumacetat in 200 ml Eisessig gelöst und nach Zugabe von 2,0 g Palladium auf Kohle (5 %ig) bei 70 °C und 4 bar hydriert. Die Wasserstoffaufnahme kommt nach 3 Stunden zum Stillstand. Der Katalysator wird abfiltriert und auf dem Filter mit Eisessig nachgewaschen. Das Filtrat wird eingedampft und der Rückstand mit Ethylacetat aufgenommen, mit Wasser gewaschen, über Magnesiumsulfat getrocknet und eingedampft. Man erhält so 15,3 g (91 % der Theorie) 4-Lilolidon als weisses kristallines Pulver, dessen sämtliche physikalischen Daten mit den Literaturangaben übereinstimmen.

## Beispiel 2

Herstellung von 5-Chlor-1,2,3-(1,2-dihydropyrrolo)-4-chinolon

In eine Lösung von 40,0 g (0,26 Mol) Phosphoroxychlorid und 19,0 g (0,26 Mol) N,N-Dimethylformamid in 150 ml Chloroform werden portionsweise 19,55 g (0,1 Mol) N-Chloracetylindolin portionsweise eingetragen. Nach beendigter Zugabe des N-Chloracetylindolins wird 24 Stunden auf Rückflusstemperatur erhitzt. Danach wird das Reaktionsgemisch durch Eindrucken von Natriumhydroxydlösung (10 %ig), Abtrennung der wässrigen Schicht und Abdampfen des Chloroforms aufgearbeitet. Es werden 12,5 g (61 % der Theorie) 5-Chlor-1,2,3-(1,2-dihydropyrrolo)-4-chinolon vom Schmelzpunkt 190-192 °C erhalten.

## Beispiel 3

Herstellung von 5-Chlor-1,2,3-(1,2-dihydropyrrolo)-4-chinolon

In eine Lösung von 14,6 g (0,2 Mol) N,N-Dimethylformamid in 70 ml 1,2-Dichlorethan werden bei 35 °C 40 g (0,4 Mol) Phosgen eingeleitet. Danach werden 19,55 g (0,1 Mol) N-Chloracetylindolin

portionsweise zugegeben und 2 Stunden bei 65 °C gerührt. Dann wird das Reaktionsgemischt auf Eis gegossen, mit Natronlauge neutralisiert, das 1,2-Dichlorethan abdestilliert, der Niederschlag filtriert und getrocknet. Man erhält so 18,9 g (92 % der Theorie) 5-Chlor-1,2,3-(1,2-dihydropyrrolo)-4-chinolon vom Schmelzpunkt 191-192 °C.

Beispiel 4

In 190 g (2,28 Mol) Thionylchlorid werden bei 25-30 °C während 1 Stunde 19,0 g (0,26 Mol) N,N-Dimethylformamid eingetropft. Anschliessend werden 19,55 g (0,1 Mol) N-Chloracetylindolin zugegeben und 3 Stunden bei 60 °C gerührt. Danach wird das überschüssige Thionylchlorid unter vermindertem Druck bei 40 °C abdestilliert, der Rückstand mit 200 g Eis verrührt und mit Natronlauge neutralisiert. Nach Filtration und Trocknen werden 8,6 g (42 % der Theorie) 5-Chlor-1,2,3-(1,2-dihydropyrrolo)-4-chinolon vom Schmelzpunkt 190-192 °C erhalten.

**Patentansprüche**

1. Verfahren zur Herstellung von 1,2,5,6-Tetrahydro-4H-pyrrolo [3,2,1-ij] chinolin-4-on, dadurch gekennzeichnet, dass man ein Halogenacetylindolin der Formel II

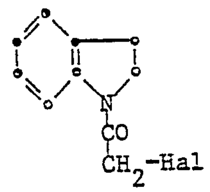

(II)

in welcher Hal Chlor oder Brom bedeutet, mit einem aus einem N,N-disubstituierten Formamid der Formel III

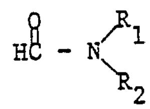

(III)

in welcher $R_1$ $C_1$-$C_4$-Alkyl oder Phenyl und $R_2$ $C_1$-$C_4$-Alkyl bedeutet, und einem Säurehalogenid gebildeten Additionsprodukt zu einem 5-Halogen-1,2,3-(1,2-dihydropyrrolo)-4-chinolon der Formel IV

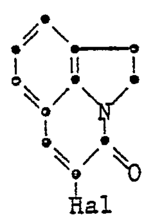

(IV)

in welcher Hal die oben angegebene Bedeutung hat, umsetzt und dieses durch katalytische Hydrierung in 4-Lilolidon der Formel I überführt.

2. Verfahren nach Anspruch 1, dadurch gekennzeichnet, dass man die Umsetzung des Halogenacetylindolins der Formel II mit dem aus einem N,N-disubstituierten Formamid der Formel III und einem Säurehalogenid gebildeten Additionsprodukt in Gegenwart eines inerten Lösungsmittels durchführt.

3. Verfahren nach Ansprüchen 1 und 2, dadurch gekennzeichnet, dass man die Umsetzung des Halogenacetylindolins der Formel II mit dem aus einem N,N-disubstituierten Formamid der Formel III und einem Säurehalogenid gebildeten Additionsprodukt in 1,2-Dichlorethan, Chloroform oder Toluol als Lösungsmittel durchführt.

4. Verfahren nach Ansprüchen 1 und 2, dadurch gekennzeichnet, dass man die Umsetzung des Halogenacetylindolins der Formel II mit dem aus einem N,N-disubstituierten Formamids der Formel III mit einem Säurehalogenid gebildeten Additionsprodukt in überschüssigem Phosphoroxychlorid als Lösungsmittel durchführt.

5. Verfahren nach Anspruch 1, dadurch gekennzeichnet, dass man als Säurehalogenid Phosphortrichlorid, Phosphortribromid, Phosphorpentachlorid, Phosphoroxychlorid, Phosphoroxybromid, Phosgen, Carbonyldibromid, Carbonyldifluorid, Oxalylchlorid, Heptachlorbuttersäurechlorid, Thionylchlorid, Thionylbromid, 2,2,2-Trichlor-1,3-dioxa-2-phosphaindan oder 2,2-Dichlor-1,3-dioxaindan verwendet.

6. Verfahren nach Anspruch 1, dadurch gekennzeichnet, dass man als Säurehalogenid Phosphoroxychlorid oder Phosgen verwendet.

7. Verfahren nach Anspruch 1, dadurch gekennzeichnet, dass man als N,N-disubstituiertes Formamid der Formel III, N,N-Dimethylformamid oder N-Methyl-N-phenylformamid verwendet.

8. Verfahren nach Anspruch 1, dadurch gekennzeichnet, dass man die Umsetzung eines Halogenacetylindolins der Formel II mit dem aus einem N,N-disubstituierten Formamid der Formel III und einem Säurehalogenid gebildeten Additionsprodukt in der Weise durchgeführt, dass man zunächst aus dem N,N-disubstituierten Formamid und dem Säurehalogenid das Additionsprodukt herstellt und danach das Halogenacetylindolin der Formel II zugibt.

9. Verfahren nach Anspruch 1, dadurch gekennzeichnet, dass man die Umsetzung eines Halogenacetylindolins der Formel II mit dem aus einem N,N-disubstituierten Formamid der Formel III und einem Säurehalogenid gebildeten Additionsprodukt in der Weise durchgeführt, dass man das aus dem N,N-disubstituierten Formamid der Formel III und dem Säurehalogenid gebildete Additionsprodukt dem Halogenacetylindolin der Formel II zufügt.

10. Verfahren nach Anspruch 1, dadurch gekennzeichnet, dass man die Umsetzung eines Halogenacetylindolins der Formel II mit dem aus einem N,N-disubstituierten Formamid der Formel III und einem Säurehalogenid gebildeten Additionsprodukt in der Weise durchführt, dass man eine Mischung eines Halogenacetylindolins der Formel II und eines N,N-disubstituierten Formamid der Formel III vorlegt und das Säurehalogenid in diese Mischung einträgt.

11. Verfahren nach Anspruch 1, dadurch gekennzeichnet, dass man die Umsetzung eines Halogenacetylindolins der Formel II mit dem aus einem N,N-disubstituierten Formamid der Formel III und einem Säurehalogenid gebildeten Additionsprodukt bei einer Temperatur zwischen 40 und 100 °C durchführt.

12. Verfahren nach Ansprüchen 1 und 11, dadurch gekennzeichnet, dass man die Umsetzung eines Halogenacetylindolins der Formel II mit dem aus einem N,N-disubstituierten Formamid der Formel III und einem Säurehalogenid gebildeten Additionsprodukt bei einer Temperatur zwischen 50 und 75 °C durchführt.

13. Verfahren nach Anspruch 1, dadurch gekennzeichnet, dass man bei Durchführung des Verfahrens in einem organischen Lösungsmittel pro Mol N-Halogenacetylindolin mindestens 2 Mol Säurehalogenid verwendet.

14. Verfahren nach Ansprüchen 1 und 13, dadurch gekennzeichnet, dass man pro Mol N-Halogenacetylindolin der Formel II 2,5-5 Mol Säurehalogenid einsetzt.

15. Verfahren nach Anspruch 1, dadurch gekennzeichnet, dass man die katalytische Hydrierung der 5-Halogen-1,2,3-(1,2-Dihydropyrrolo)-4-chinolone der Formel IV in Gegenwart eines Edelmetalls der Gruppe 8 des periodischen Systems durchführt.

16. Verfahren nach Ansprüchen 1 und 14, dadurch gekennzeichnet, dass man die katalytische Hydrierung der 5-Halogen-1,2,3-(1,2-dihydropyrrolo)-4-chinolone der Formel IV in Gegenwart von Nickel, Palladium oder Platin als Katalysator durchführt.

17. Verfahren nach Anspruch 1, dadurch gekennzeichnet, dass man die katalytische Hydrierung der 5-Halogen-1,2,3-(1,2-dihydropyrrolo)-4-chinolone der Formel IV in einem inerten organischen Lösungsmittel und in Gegenwart einer Base durchführt.

18. Verfahren nach Anspruch 1, dadurch gekennzeichnet, dass man die katalytische Hydrierung der 5-Halogen-1,2,3-(1,2-dihydropyrrolo)-4-chinolone der Formel IV bei einem Druck von 1-20 bar und einer Temperatur zwischen Raumtemperatur und 130 °C durchführt.

19. Verfahren nach Anspruch 1, dadurch gekennzeichnet, dass man die katalytische Hydrierung der 5-Halogen-1,2,3-(1,2-dihydropyrrolo)-4-chinolone der Formel IV bei einem Druck von 3-5 bar und einer Temperatur von 40-75 °C durchführt.

20. 5-Halogen-1,2,3-(1,2-dihydropyrrolo)-4-chinolone der Formel IV

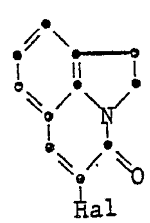

(IV)

in welcher Hal Chlor oder Brom bedeutet.

## Claims

1. A process for the preparation of 1,2,5,6-tetrahydro-4H-pyrrolo [3,2,1-ij] quinolin-4-one, which process comprises reacting a haloacetylindoline of the formula II

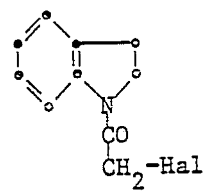

(II)

in which Hal is chlorine or bromine, with an addition porduct formed from an N,N-disubstitued formanide of the formula III

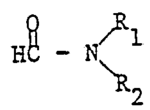

(III)

in which $R_1$ is $C_1$-$C_4$-alkyl or phenyl, and $R_2$ is $C_1$-$C_4$-alkyl, and an acid halide to give a 5-halo-1,2,3-(1,2-dihydropyrrolo)-4-quinolone of the formula IV

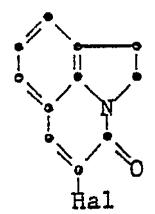

(IV)

in which Hal is as defined above ; and then converting this compound, by catalytic hydrogenation, into a 4-lilolidone of the formula I.

2. A process according to claim 1, wherein the reaction of the haloacetylindoline of the formula II with the addition product formed from an N,N-disubstituted formamide of the formula III and an acid halide is performed in the presence of an inert solvent.

3. A process according to either of claims 1 or 2, wherein the reaction of the haloacetylindoline of the formula II with the addition product formed from an N,N-disubstituted formamide of the formula III and an acid halide is performed in 1,2-dichloroethane, chloroform or toluene as solvent.

4. A process according to either of claims 1 or 2, wherein the reaction of the haloacetylindoline of the formula II with the addition product formed from an N,N-disubstituted formamide of the formula III and an acid halide is performed in excess phosphorus oxychloride as solvent.

5. A process according to claim 1, wherein the acid halide is selected from the group comprising : phosphorus trichloride, phosphorus tribromide, phosphorus pentachloride, phosphorus oxychloride, phosphorus oxybromide, phosgene, carbonyl dibromide, carbonyl difluoride, oxalyl chloride, heptachlorobutyryl chloride, thionyl chloride, thionyl bromide, 2,2,2-trichloro-1,3-dioxa-2-phosphaindane or 2,2-dichloro-1,3-dioxaindane.

6. A process according to claim 1 wherein the acid halide is phosphorus oxychloride or phosgene.

7. A process according to claim 1, wherein the N,N-disubstituted formamide of the formula III is N,N-dimethylformamide or N-methyl-N-phenylformamide.

8. A process according to claim 1, wherein the reaction of a haloacetylindoline of the formula II with the addition product formed from an N,N-disubstituted formamide of the formula III and an acid halide is performed by firstly producing the addition product from the N,N-disubstituted formamide and the acid halide, and afterwards adding the haloacetylindoline of the formula II.

9. A process according to claim 1, wherein the reaction of a haloacetylindoline of the formula II with the addition product formed from an N,N-disubstituted formamide of the formula III and an acid halide is performed by adding the addition product formed from the N,N-disubstituted formamide of the formula III and the acid halide to the haloacetylindoline of the formula II.

10. A process according to claim 1, wherein the reaction of a haloacetylindoline of the formula II with the addition product formed from an N,N-disubstituted formamide of the formula III and an acid halide is performed by charging a reaction vessel with a mixture of a haloacetylindoline of the formula II and an N,N-disubstituted formamide of the formula III, and then introducing the acid halide into this mixture.

11. A process according to claim 1, wherein the reaction of a haloacetylindoline of the formula II with the addition formed from an N,N-disubstituted formamide of the formula III and an acid halide is performed at a temperature in the range from 40 to 100 °C.

12. A process according to either of claims 1 or 11, wherein the reaction of a haloacetylindoline of the formula II with the addition product formed from an N,N-disubstituted formamide of the formula III and an acid halide is performed at a temperature in the range from 50 to 75 °C.

13. A process according to claim 1, which, when the process is performed in an organic solvent, comprises the use of at least 2 moles of acid halide per mole of N-haloacetylindoline.

14. A process according to either of claims 1 or 13, which comprises the use of 2.5 - 5 moles of acid halide per mole of N-haloacetylindoline of the formula II.

15. A process according to claim 1, wherein the catalytic hydrogenation of the 5-halo-1,2,3-(1,2-dihydropyrrolo)-4-quinolones of the formula IV is performed in the presence of a noble metal of the group VIII of the periodic system.

16. A process according to either of claims 1 or 14, wherein the catalytic hydrogenation of the 5-halo-1,2,3-(1,2-dihydropyrrolo)-4-quinolones of the formula IV is performed in the presence of nickel, palladium or platinum as catalyst.

17. A process according to claim 1, wherein the catalytic hydrogenation of the 5-halo-1,2,3-(1,2-dihydropyrrolo)-4-quinolones of the formula IV is performed in an inert organic solvent and in the presence of a base.

18. A process according to claim 1, wherein the catalytic hydrogenation of the 5-halo-1,2,3-(1,2-dihydropyrrolo)-4-quinolones of the formula IV is performed under a pressure of 1-20 bar and at a temperature in the range from room temperature to 130 °C.

19. A process according to claim 1, wherein the catalytic hydrogenation of the 5-halo-1,2,3-(1,2-dihydropyrrolo)-4-quinolones of the formula IV is performed under a pressure of 3-5 bar and at temperature in the range from 40 to 75 °C.

20. A 5-halo-1,2,3-(1,2-dihydropyrrolo)-4-quinolone of the formula IV

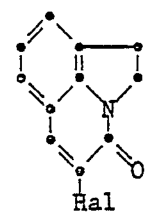

(IV)

in which Hal is chlorine or bromine.

**Revendications**

1. Procédé de préparation de la 1,2,5,6-tétrahydro-4H-pyrrolo [3,2,1-i] quinoléine-4-one, caractérisé en ce que l'on fait réagir une halogénoacétylindoline de formule II

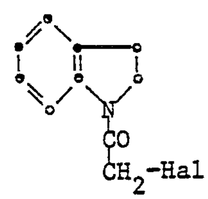

(II)

dans laquelle Hal représente le chlore ou le brome, avec un produit d'addition formé à partir d'un formamide N,N-disubstitué de formule III :

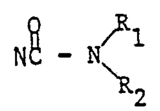

(III)

dans laquelle $R_1$ représente un groupe alkyle en $C_1$-$C_4$ ou phényle et $R_2$ représente un groupe alkyle en $C_1$-$C_4$, et d'un halogénure d'acide, ce qui donne une 5-halogéno-1,2,3-(1,2-dihydropyrrolo)-4-quinolone de formule IV :

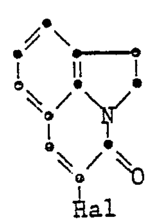

(IV)

dans laquelle Hal a les significations indiquées ci-dessus, qu'on convertit par hydrogénation catalytique en la 4-lilolidone de formule I.

2. Procédé selon la revendication 1, caractérisé en ce que la réaction de l'halogénoacétylindoline de formule II avec le produit d'addition formé à partir d'un formamide N,N-disubstitué de formule III et d'un halogénure d'acide est effectuée en présence d'un solvant inerte.

3. Procédé selon les revendications 1 et 2, caractérisé en ce que la réaction de l'halogénoacétylindoline de formule II avec le produit d'addition formé à partir du formamide N,N-disubstitué de formule III et

d'un halogénure d'acide est effectuée dans un solvant consistant en le 1,2-dichloréthane, le chloroforme ou le toluène.

4. Procédé selon les revendications 1 et 2, caractérisé en ce que la réaction de l'halogénoacétylindoline de formule II avec le produit d'addition formé à partir d'un formamide N,N-disubstitué de formule III et d'un halogénure d'acide est effectuée dans un solvant qui consiste en un excès d'oxychlorure de phosphore.

5. Procédé selon la revendication 1, caractérisé en ce que l'on utilise en tant qu'halogénure d'acide le trichlorure de phosphore, le tribromure de phosphore, le pentachlorure de phosphore, l'oxychlorure de phosphore, l'oxybromure de phosphore, le phosgène, le dibromure de carbonyle, le difluorure de carbonyle, le chorure d'oxalyle, le chlorure de l'acide heptachlorobutyrique, le chlorure de thionyle, le bromure de thionyle, le 2,2,2-trichloro-1,3-dioxa-2-phosphaindane ou le 2,2-dichloro-1,3-dioxa-indane.

6. Procédé selon la revendication 1, caractérisé en ce que l'halogénure d'acide utilisé est l'oxychlorure de phosphore ou le phosgène.

7. Procédé selon la revendication 1, caractérisé en ce que l'on utilise en tant que formamide N,N-disubstitué de formule III, le N,N-diméthylformamide ou le N-méthyl-N-phénylformamide.

8. Procédé selon la revendication 1, caractérisé en ce que la réaction d'une halogénoacétylindoline de formule II avec le produit d'addition formé à partir du formamide N,N-disubstitué de formule III et d'un halogénure d'acide est effectuée en préparant d'abord, à partir du formamide N,N-disubstitué et de l'halogénure d'acide, le produit d'addition auquel on ajoute ensuite l'halogénoacétylindoline de formule II.

9. Procédé selon la revendication 1, caractérisé en ce que la réaction d'une halogénoacétylindoline de formule II avec le produit d'addition formé à partir d'un formamide N,N-disubstitué de formule III et d'un halogénure d'acide est effectuée en ajoutant le produit d'addition formé à partir du formamide N,N-disubstitué de formule III et de l'halogénure d'acide à l'halogénoacétylindoline de formule II.

10. Procédé selon la revendication 1, caractérisé en ce que la réaction d'une halogénoacétylindoline de formule II avec le produit d'addition formé à partir d'un formamide N,N-disubstitué de formule III et d'un halogénure d'acide est effectuée en préparant un mélange d'une halogénoacétylindoline de formule II et d'un formamide N,N-disubstitué de formule III et en introduisant l'halogénure d'acide dans ce mélange.

11. Procédé selon la revendication 1, caractérisé en ce que la réaction d'une halogénoacétylindoline de formule II avec le produit d'addition formé à partir d'un formamide N,N-disubstitué de formule III et d'un halogénure d'acide est effectuée à une température de 40 à 100 °C.

12. Procédé selon les revendications 1 et 11, caractérisé en ce que la réaction d'une halogénoacétylindoline de formule II avec le produit d'addition formé à partir d'un formamide N,N-disubstitué de formule III et d'un halogénure d'acide est effectuée à une température de 50 à 75 °C.

13. Procédé selon la revendication 1, caractérisé en ce que, lorsque le procédé est mis en œuvre dans un solvant organique, on utilise au moins 2 moles de l'halogénure d'acide par mole de la N-halogénoacétylindoline.

14. Procédé selon les revendications 1 et 13, caractérisé en ce que l'on utilise de 2,5 à 5 moles d'halogénure d'acide par mole de la N-halogénoacétylindoline de formule II.

15. Procédé selon la revendication 1, caractérisé en ce que l'hydrogénation catalytique des 5-halogéno-1,2,3-(1,2-dihydropyrrolo)-4-quinolones de formule IV est effectuée en présence d'un métal noble du 8$^{ème}$ groupe de la Classification Périodique.

16. Procédé selon les revendications 1 et 14, caractérisé en ce que l'hydrogénation catalytique des 5-halogéno-1,2,3-(1,2-dihydropyrrolo)-4-quinolones de formule IV est effectuée en présence de nickel, de palladium ou de platine servant de catalyseurs.

17. Procédé selon la revendication 1, caractérisé en ce que l'hydrogénation catalytique des 5-halogéno-1,2,3-(1,2-dihydropyrrolo)-4-quinolones de formule IV est effectuée dans un solvant organique inerte et en présence d'une base.

18. Procédé selon la revendication 1, caractérisé en ce que l'hydrogénation catalytique des 5-halogéno-1,2,3-(1,2-dihydropyrrolo)-4-quinolones de formule IV est effectuée sous une pression de 1 à 20 bars et à une température allant de la température ambiante jusqu'à 130 °C.

19. Procédé selon la revendication 1, caractérisé en ce que l'hydrogénation catalytique des 5-halogéno-1,2,3-(1,2-dihydropyrrolo)-4-quinolones de formule IV est effectuée sous une pression de 3 à 5 bars et à une température de 40 à 75 °C.

20. Les 5-halogéno-1,2,3-(1,2-dihydropyrrolo)-4-quinolones de formule IV :

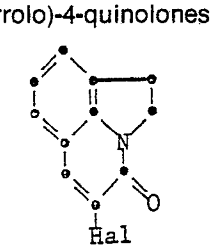

(IV)

dans laquelle Hal représente le chlore ou le brome.